(19) Europäisches Patentamt
European Patent Office
Office européen des brevets

(11) Publication number: **0 018 134**
**B1**

(12) # EUROPEAN PATENT SPECIFICATION

(45) Date of publication of patent specification: **14.03.84**

(51) Int. Cl.³: **C 07 D 213/75,**
C 07 D 213/80,
C 07 D 413/12,
A 61 K 31/44

(21) Application number: **80301050.3**

(22) Date of filing: **02.04.80**

(54) **Dihydropyridine derivatives, processes for their preparation and pharmaceutical compositions containing them.**

(30) Priority: **21.04.79 GB 7913950**

(43) Date of publication of application:
**29.10.80 Bulletin 80/22**

(45) Publication of the grant of the patent:
**14.03.84 Bulletin 84/11**

(84) Designated Contracting States:
**CH DE FR GB IT LI NL**

(56) References cited:
**EP - A - 0 000 816**
**EP - A - 0 009 362**
**BE - A - 852 565**
**US - A - 3 933 836**

(73) Proprietor: **BEECHAM GROUP PLC**
**Beecham House Great West Road**
**Brentford Middlesex (GB)**

(72) Inventor: **Cantello, Barrie Christian Charles**
**8 Woodside Way**
**Redhill Surrey (GB)**

(74) Representative: **Russell, Brian John et al,**
**European Patent Attorney Beecham**
**Pharmaceuticals Great Burgh Yew Tree Bottom**
**Road**
**Epsom Surrey, KT18 5XQ (GB)**

**EP 0 018 134 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

Dihydropyridine derivatives, processes for their preparation, and pharmaceutical compositions containing them

This invention relates to a class of novel dihydropyridine derivatives which are useful in the treatment of diabetes. The invention also relates to a process for their preparation and to pharmaceutical compositions containing them.

The compound of formula (I):

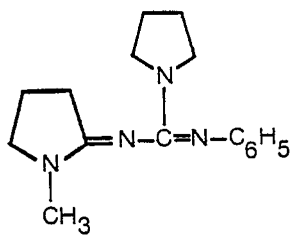

(I)

has been reported to be useful in the treatment of diabetes in Belgian Patent No. 852,565 and in Diabetes, *27*, 856 and 868 (1978).

European Patent Application No. 79301860.7 (Published No. 9362) which falls within the terms of Article 54(3) EPC, discloses *inter alia* compounds of the general formula (IA):

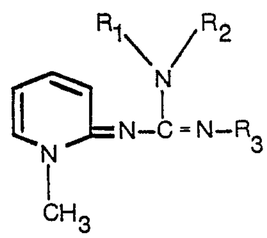

(IA)

wherein:

$R_1$ is methyl or ethyl;

$R_2$ is lower alkyl, cyclopentyl, cyclohexyl or benzyl;

or 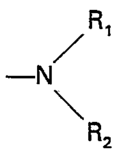 taken together represents 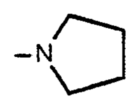, 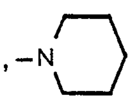, or 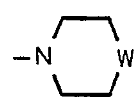

wherein:

W is oxygen, sulphur, N-lower alkyl or N-aryl; and

$R_3$ is alkyl having from 4 to 10 carbons; phenyl; methylenedioxyphenyl; phenyl substituted with from 1 to 3 substituents consisting of halo, lower alkyl, or lower alkoxy; or phenyl substituted with hydroxy, benzyloxy, nitro; trifluoromethyl or methylthio; naphthyl; cyclopentyl; cyclohexyl; exo-2-norbornyl; endo-2-norbornyl; 1-adamantyl; arylalkyl in which the aryl function is phenyl and the alkyl function has from 1 to 4 carbons; diphenylalkyl in which the alkyl function has from 1 to 2 carbons.

These compounds are said to have hypoglycaemic properties.

U.S. Patent No. 3,933,836 discloses compounds of the general formula (IB):

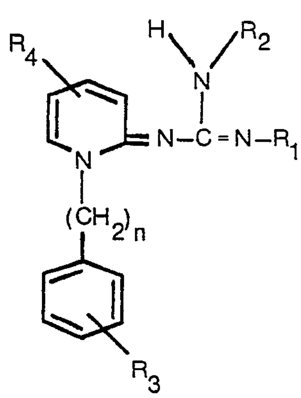

(IB)

wherein:

$R_1$ and $R_2$ are alkyl, cycloalkyl, aryl or aralkyl;

$R_3$ is hydrogen, halogen, trifluoromethyl, alkoxy, aryloxy or dialkylamidosulphonyl;

$R_4$ is hydrogen, halogen, alkyl, alkoxy or aryl; and

n is 1 or 2.

These compounds are said to be CNS stimulants, but there is no disclosure of them having hypoglycaemic activity.

We have now found a novel class of dihydropyridylidene compounds which have hypoglycaemic activity.

Accordingly the present invention provides a compound of formula (II) or a pharmaceutically acceptable quaternary ammonium or acid addition salt thereof:

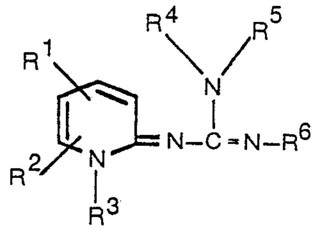 . (II)

wherein $R^1$ and $R^2$ are substituents attached to any of the carbon atoms of the dihydropyridine ring and represent hydrogen, $C_{1-6}$ alkyl, halogen, carbo-$C_{1-6}$ alkoxy or carboxy.

$R^3$ represents $C_{1-6}$ alkyl;

$R^4$ represents hydrogen or $C_{1-6}$ alkyl;

$R^5$ represents $C_{1-6}$ alkyl, phenyl optionally substituted with up to 3 groups selected from halogen $C_{1-6}$ alkyl, and $C_{1-6}$ alkoxy; or benzyl optionally substituted with up to 3 groups selected from halogen, $C_{1-6}$ alkyl and $C_{1-6}$ alkoxy; or $R^4$ and $R^5$ together represent the remaining members of a 5- or 6-membered ring optionally containing an oxygen, sulphur or additional nitrogen atom and being optionally substituted with $C_{1-6}$ alkyl; and

$R^6$ represents $C_{1-6}$ alkyl, phenyl, optionally substituted with up to 3 groups selected from halogen, nitro-, amino-, or trifluoromethyl, $C_{1-6}$ alkyl and $C_{1-6}$ alkoxy; or benzyl optionally substituted with up to 3 groups selected from halogen, $C_{1-6}$ alkyl and $C_{1-6}$ alkoxy, with the proviso that the compounds and acid addition salts thereof wherein, simultaneously, $R^1$ and $R^2$ are both hydrogen, $R^3$ is methyl, $R^4$ is methyl or ethyl, $R^5$ is $C_{1-4}$ alkyl or benzyl,

or 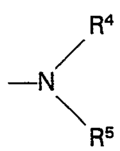 taken together represents 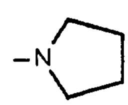 , 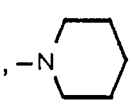 , or 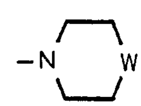

wherein W is oxygen, sulphur or N—$C_{1-4}$ alkyl, are disclaimed.

Suitable acid addition salts of compound (II) include inorganic salts such as the sulphate, nitrate, phosphate, and borate, hydrohalides such as the hydrochloride, hydrobromide and hydroiodide, and organic acid addition salts such as acetate, oxalate, tartrate, maleate, citrate, succinate, benzoate, ascorbate, methanesulphonate and p-toluenesulphonate.

Preferred salts are hydrohalide salts.

Suitable quaternary salts are the $C_{1-6}$ alkyl halides, in particular the methylhalide, such as the methyliodide salt.

Examples of suitable $C_{1-6}$ alkyl groups which $R^1$ to $R^6$ may represent include methyl, ethyl, *n*- and *iso*- propyl, and *n*-, *sec*-, *iso*- and *tert*-butyl.

Suitable substituents for the phenyl and benzyl groups for $R^5$ and $R^6$ include *ortho*-, *meta*- and *para*-methyl, methoxy, chloro, bromo, fluoro, nitro and amino (the last two substituents being only for $R^6$).

Suitably $R^1$ and $R^2$ represent hydrogen, methyl or halogen particularly hydrogen.

Suitably $R^3$ is methyl, ethyl or *n*-propyl, but preferably $R^3$ is methyl.

Suitably $R^4$ is hydrogen, methyl, ethyl, or *n*-propyl, and $R^5$ represents methyl, ethyl, *n*-propyl, phenyl or benzyl. Preferably $R^4$ and $R^5$ are both ethyl. When $R^4$ and $R^5$ complete a ring, suitable such rings include pyrrolidine, piperidine, morpholine, thiamorpholine, piperazine and 4-methylpiperazine rings. Preferably $R^4$ and $R^5$ complete a morpholine ring.

Suitably $R^6$ is methyl, ethyl, *n*-propyl or phenyl, but preferably $R^6$ is phenyl or the herefore defined substituted phenyl.

One sub-group of compounds falling within the scope of the compounds of formula (II) and

subject to the same disclaimer thereto, comprises compounds of formula (III) and pharmaceutically acceptable quaternary ammonium or acid addition salts thereof:

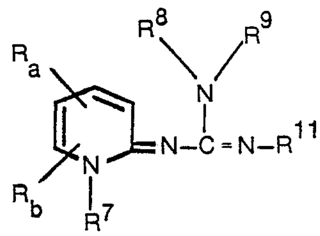

(III)

wherein $R_a$ and $R_b$ are the same as $R^1$ and $R^2$ respectively as defined in formula (II), $R^7$ represents $C_{1-6}$ alkyl, preferably methyl,

$R^8$ represents hydrogen or $C_{1-6}$ alkyl;

$R^9$ represents $C_{1-6}$ alkyl or benzyl; or $R^8$ and $R^9$ together form the remaining members of a pyrrolidine, thiamorpholine or morpholine ring, and

$R^{11}$ represents phenyl optionally substituted with up to 3 groups selected from $C_{1-6}$ alkyl, halogen, or nitro or amino.

Preferably, $R^8$ and $R^9$ are both ethyl, and when taken together form a morpholine ring.

Examples of compounds of formula (II) include the following:

N - (1,2 - dihydro - 1 - methyl - 2 - pyridylidene) - N' - phenyl - 4 - (2,6 - dimethylmorpholine)-carboxamidine, N - benzyl - N' - (1,2 - dihydro - 1 - methyl - 2 - pyridylidene) - N'' - phenylguanidine, N,N - diethyl - N' - (1,2 - dihydro - 1,3 - dimethyl - 2 - pyridylidene) - N'' - phenylguanidine hydroiodide, N - (1,2 - dihydro - 1,4 dimethyl - 2 - pyridylidene) - N' - phenyl - 4 - morpholinecarboxamidine hydroiodide, N - (5 - bromo - 1,2 - dihydro - 1 - methyl - 2 - pyridylidene) - N' - phenyl - 4 - morpholinecarboxamidine hydroiodide, N - (5 - carbomethoxy - 1,2 - dihydro - 1 - methyl-2 - pyridylidene) - N' - phenyl - 4 - morpholinecarboxamidine hydroiodide, N - (1,2 - dihydro - 1,5-dimethyl - 2 - pyridylidene) - N' - phenyl - 4 - morpholinecarboxamidine.

Compounds of formula (II) may be prepared by reacting a compound of formula (IV) or a salt thereof:

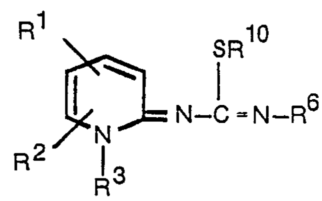

(IV)

wherein $R^1$, $R^2$, $R^3$ and $R^6$ are as defined with respect to formula (II) above, and $R^{10}$ represents $C_{1-6}$ alkyl; with an amine of formula $R^4R^5NH$, wherein $R^4$ and $R^5$ are as defined with reference to formula (II) above and thereafter where desired converting a free base of formula (II) so obtained into a pharmaceutically acceptable salt or converting a salt of a compound of formula (II) so obtained into the free base.

The reaction is conveniently carried out in polar organic solvent, the choice of which is not critical to the success of the reaction provided that it forms a homogeneous solution of the reagent and is substantially inert to the reagent and product. Convenient solvents include alkanols such as *iso*-propanol and alkanoic acids such as glacial acetic acid.

The reaction is generally carried out at a moderate temperature i.e. greater than room temperature, the reflux temperature of the solvent being selected for convenience.

The period for which the reaction is allowed to proceed depends upon the particular starting materials employed. The course of the reaction may be followed by conventional methods such as thin layer chromatography and terminated when an optimum quantity of product is present in the reaction mixture. However, in general we have found that it is convenient to leave the reaction mixture to reflux overnight.

Examples of $C_{1-6}$ alkyl groups which $R^{10}$ may represent include methyl, ethyl, *n*-propyl, or *n*-butyl, but preferably $R^{10}$ represents methyl.

The intermediates of formula (IV) may be prepared by the route shown in the following scheme:

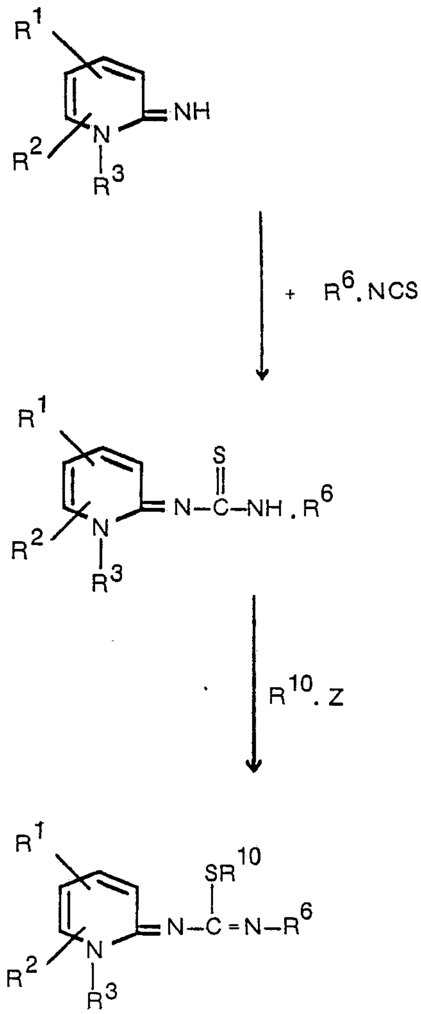


(V)

(VI)

(IV)

Thus, intermediates (IV) are prepared by alkylation of a thiourea (VI) using an alkylating agent $R^{10}$.Z wherein $R^{10}$ is as defined with reference to formula (IV) and Z is a leaving group such as chloride, bromide or iodide. Suitably the reaction is carried out in a polar organic solvent, the choice of which is not critical. Suitable solvents include lower alkanones. The reaction is suitably carried out at the boiling point of the solvent.

The thiourea (VI) is in turn prepared by reacting an *iso*-thiocyanate $R^6$.NCS with a corresponding imino compound (V), where $R^1$, $R^2$, $R^3$ and $R^6$ are as defined with reference to formula (II). This reaction is carried out in a solvent such as ethanol, toluene, benzene, dioxane or tetrahydrofuran. The reaction is carried out at non-extreme temperatures i.e. up to and including the reflux temperature of the solvent.

Compounds of formula (II) may also be prepared by reacting a compound of formula (VII).

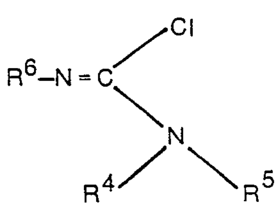

(VII)

wherein $R^4$, $R^5$ and $R^6$ are as defined with respect to formula (II) above; with an imino compound (V), where $R^1$, $R^2$ and $R^3$ are as defined with respect to formula (II) above and thereafter where desired converting a free base of formula (II) so obtained into a pharmaceutically acceptable salt or converting a salt of a compound of formula (II) so obtained into the free base.

The reaction is conveniently carried out in a non-hydroxylic solvent system such as an ether, chlorinated hydrocarbon or a mixture thereof. Suitable solvent systems include mixtures of diethyl ether and chloroform. The reaction is suitably carried out at ambient temperature. The period for which the reaction is allowed to proceed may be determined by methods as described hereinbefore; however, we have found it convenient to leave the reaction mixture to stand overnight.

The intermediates of formula (VII) may be prepared by reaction of an isocyanide dichloride of formula: $R^6$—N=$CCl_2$ wherein $R^6$ is as defined with respect to formula (II) above; with an amine of formula $R^4R^5NH$, wherein $R^4$ and $R^5$ are as defined with reference to formula (II) above. Suitably the reaction is carried out in ethereal solvent such as diethyl ether or tetrahydrofuran. The reaction is suitably carried out at ambient temperature. The period for which the reaction is allowed to proceed may be determined by methods as described hereinbefore; however, we have found a two hour reaction time to be sufficient.

In order to put the compounds (II) to use as medicinal agents for the treatment of diabetes, they are presented as pharmaceutical compositions in a variety of dosage forms. This invention therefore also includes a pharmaceutical composition which comprises a compound of formula (II) together with a pharmaceutically acceptable carrier or excipient.

The compositions may be formulated for administration by any route, although an oral administration is preferred. The compositions may be in the form of tablets, capsules, powders, granules, lozenges, or liquid preparations, such as oral or sterile parenteral solutions or suspensions.

Tablets and capsules for oral administration may be in unit dose presentation form, and may contain conventional excipients such as binding agents, for example syrup, acacia, gelatin, sorbitol, tragacanth, or polyvinylpyrrolidone; fillers, for example lactose, maize-starch, calcium phosphate, sorbitol or glycine; tabletting lubricants, for example magnesium stearate, talc, polyethylene glycol, or silica; disintegrants, for example potato starch; or acceptable wetting agents such as sodium lauryl sulphate. The tablets may be coated according to methods well known in normal pharmaceutical practice. Oral liquid preparations may be in the form of, for example, aqueous or oily suspensions, solutions, emulsions, syrups, or elixirs, or may be presented as a dry product for reconstitution with water or other suitable vehicle before use. Such liquid preparations may contain conventional additives such as suspending agents, for example sorbitol, syrup, methyl cellulose, gelatin, hydroxyethylcellulose, carboxy-methyl cellulose, aluminium stearate gel or hydrogenated edible fats, emulsifying agents, for example lecithin, sorbitan monooleate, or acacia; non-aqueous vehicles (which may include edible oils), for example almond oil, fractionated coconut oil, oily esters such as glycerine, propylene glycol, or ethyl alcohol; preservatives, for example methyl or propyl *p*-hydroxybenzoate or sorbic acid, and if desired conventional flavouring or colouring agents. The compound may also if desired be incorporated in a foodstuff, for example in the form of a biscuit.

For parenteral administration, fluid unit dosage forms are prepared utilizing the compound and a sterile vehicle, and concentration used, can be either suspended or dissolved in the vehicle. In preparing solutions the compound can be dissolved in water for injection and filter sterilized before filling into a suitable vial or ampoule and sealing. Advantageously, adjuvants such as local anaesthetic, preservative and buffering agents can be dissolved in the vehicle. To enhance the stability, the composition can be frozen after filling into the vial and the water removed under vacuum. Parenteral suspensions are prepared in substantially the same manner except that the compound is suspended in the vehicle instead of being dissolved and sterilization cannot be accomplished by filtration. The compound can be sterilized by exposure to ethylene oxide before suspending in the sterile vehicle. Advantageously, a surfactant or wetting agent is included in the composition to facilitate uniform distribution of the compound.

The compositions may contain from 0.1% to 99% by weight, preferably from 10—60% by weight, of the active material, depending on the method of administration. The dosage employed for adult treatment will of course depend on the dose-response characteristics of the particular active ingredient but will normally be in the range 0.1 to 150 mg/kg/day.

The following Examples 3, 4 and 10 to 14 illustrate the preparation of a number of compounds of this invention. Examples 1, 2, 5 to 9 and 15 to 17 illustrate the preparation of comparative examples, which do not form part of the present invention.

## Example 1

*N - (1,2 - Dihydro - 1 - methyl - 2 - pyridylidene) - N' - phenyl - 1 -pyrrolidinecaroxamidine hydroiodide*

a) *1 - (1,2 - Dihydro - 1 - methyl - 2 - pyridylidene) - 3 - phenyl - 2 - thiourea*

Sodium methoxide (0.71 g) was added to 1,2-dihydro-2-imino-1-methylpyridine hydroiodide (3.1 g) in ethanol (30 ml) and heated to reflux when phenylisocyanate (1.96 g) in ethanol (20 ml) was added over 5 minutes. The resultant mixture was heated under reflux, with stirring, for 50 minutes, cooled in ice, filtered, washed well with ethanol and dried to give the product, mpt 186.5—188°, of analytical purity. Recrystallisation from ethanol gave a sample mpt 186—187°.

b) *3 - (1,2 - Dihydro - 1 - methyl - 2 - pyridylidene) - 2 - methyl - 1 - phenyl - 2 - thiopseudourea hydroiodide*

A mixture of 1 - (1,2, - dihydro - 1 - methyl - 2 - pyridylidene) - 3 - phenyl - 2 - thiourea (15.5 g), methyl iodide (11.93 g) and acetone (175 ml) was heated under reflux, with stirring, for 40 minutes, cooled in ice and filtered to give the analytically pure product, mpt 150—151°.

c) *N - (1,2 - Dihydro - 1 -methyl - 2 - pyridylidene) - N'phenyl - 1 - pyrrolidenecarboxamidine hydroiodide*

A mixture of 3 - (1,2 - dihydro - 1 - methyl - 2 - pyridylidene) - 2 - methyl - 1 - phenyl - 2 - thiopseudourea hydroiodide (2.55 g) and pyrrolidine (3.24 g) in dry *iso*-propanol (60 ml) was heated under reflux, with stirring, for 20 hours, cooled in ice, filtered, washed well with *iso*-propanol then diethyl ether to give the analytically pure product as its hydroiodide salt, mpt 239—240°.

Examples 2—4

By an analogous procedure to that described in Example 1(c), the following products were obtained by reaction of 3-(1,2-dihydro-1-methyl-2-pyridylidene)-2-methyl-1-phenyl-2-thiopseudourea hydroiodide, described in Example 1(b), with the appropriate amine:

Example 2

N-(1,2-dihydro-1-methyl-2-pyridylidene)-N'-phenyl-4-morpholinecarboxamidine hydroiodide, mpt 213.5—215°.

Example 3

N-(1,2-dihydro-1-methyl-2-pyridylidene)-N'-phenyl-4-(2,6-dimethylmorpholine)-carboxamidine, mpt 142—4°.

Example 4

N-benzyl-N'-(1,2-dihydro-1-methyl-2-pyridylidene)-N''-phenylguanidine as an oil which was purified by column chromatography on silica using 7% of (33% dimethylamine in ethanol) in dichloromethane.

Examples 5—13

The following thioureas were prepared, by an analogous procedure to that described in Example 1(a), from the appropriate imine and isothiocyanate:

1-(1,2-dihydro-1-methyl-2-pyridylidene)-3-(4-methylphenyl)-2-thiourea.

3-(4-chlorophenyl)-1-(1,2-dihydro-1-methyl-2-pyridylidene)-2-thiourea, mpt 164—6°.

1-(1,2-dihydro-1-methyl-2-pyridylidene)-3-(4-fluorophenyl)-2-thiourea, mpt 179—80°.

1-(1,2-dihydro-1-methyl-2-pyridylidene)-3-(4-nitrophenyl)-2-thiourea, mpt 204—8°.

1-(1,2-dihydro-1,3-dimethyl-2-pyridylidene)-3-phenyl-2-thiourea, mpt 202.5—203.5°.

1-(1,2-dihydro-1,4-dimethyl-2-pyridylidene)-3-phenyl-2-thiourea, mpt 207—9°.

1-(5-bromo-1,2-dihydro-1-methyl-2-pyridylidene)-3-phenyl-2-thiourea, mpt 183—6° (chloroform-diethyl ether).

1-(5-carbomethoxy-1,2-dihydro-1-methyl-2-pyridylidene)-3-phenyl-2-thiourea, mpt 176—8°.

Reaction of these thioureas with methyl iodide by an analogous procedure to that described in Example 1(b) gave the following 2-methyl-2-thiopseudoureas:

3-(1,2-dihydro-1-methyl-2-pyridylidene-2-methyl-1-(4-methylphenyl)-2-thiopseudourea hydroiodide, mpt 147—50°.

1-(4-chlorophenyl)-3-(1,2-dihydro-1-methyl-2-pyridylidene)-2-methyl-2-thiopseudourea hydroiodide, mpt 163—4° (isopropanol).

3-(1,2-dihydro-1-methyl-2-pyridylidene)-1-(4-fluorophenyl)-2-methyl-2-thiopseudourea hydroiodide, mpt 114—5.5°.

3-(1,2-dihydro-1-methyl-2-pyridylidene)-2-methyl-1-(4-nitrophenyl)-2-thiopseudourea hydroiodide, mpt 173—6°.

3-(1,2-dihydro-1,3-dimethyl-2-pyridylidene)-2-methyl-1-phenyl-2-thiopseudourea hydroiodide.

3-(1,2-dihydro-1,4-dimethyl-2-pyridylidene)-2-methyl-1-phenyl-2-thiopseudourea hydroiodide, mpt 168—72° (methanol-diethyl ether).

3-(5-bromo-1,2-dihydro-1-methyl-2-pyridylidene)-2-methyl-1-phenyl-2-thiopseudourea hydroiodide, mpt 184—6°.

3-(5-carbomethoxy-1,2-dihydro-1-methyl-2-pyridylidene)-2-methyl-1-phenyl-2-thiopseudourea hydroiodide, mpt 168—9°.

Reaction of the appropriate 2-methyl-2-thiopseudourea hydroiodide and amine gave the following guanidines by an analogous procedure ot that described in Example 1(c):

Example 5

N-(1,2-dihydro-1-methyl-2-pyridylidene)-N'-(4-methylphenyl)-4-morpholinecarboxamidine hydroiodide, mpt 206—7° (ethanol-diethyl ether).

Example 6

N'-(4-chlorophenyl)-N-(1,2-dihydro-1-methyl-2-pyridylidene)-4-morpholinecarboxamidine hydroiodide, mpt 207—9° (methanol-diethyl ether).

Example 7

N-(1,2-dihydro-1-methyl-2-pyridylidene)-N'-(4-fluorophenyl)-4-morpholinecarboxamidine hydroiodide, mpt 171—3°.

Example 8

N,N-diethyl-N'-(1,2-dihydro-1-methyl-2-pyridylidene)-N''-(4-fluorophenyl)guanidine hydroiodide, mpt 129.5—130.5° (methanol-diethyl ether).

Example 9

N-(1,2-dihydro-1-methyl-2-pyridylidene)-N'-(4-nitrophenyl)-4-morpholinecarboxamidine hydroiodide, mpt 221—3°.

Example 10

N,N-diethyl-N'-(1,2-dihydro-1,3-dimethyl-2-pyridylidene)-N''-phenylguanidine hydroiodide, mpt 215—6°, (methanol-diethyl ether).

Example 11

N-(1,2-dihydro-1,4-dimethyl-2-pyridylidene)-N'-phenyl-4-morpholinecarboxamidine hydroiodide, mpt 181—2° (methanol-diethyl ether).

Example 12

N-(5-bromo-1,2-dihydro-1-methyl-2-pyridylidene)-N'-phenyl-4-morpholinecarboxamidine hydroiodide, mpt 226—8°.

Example 13

N-(5-carbomethoxy-1,2-dihydro-1-methyl-2-pyridylidene)-N'-phenyl-4-morpholinecarboxamidine hydroiodide, mpt 214—5° (methanol-diethyl ether).

Example 14

*N-(1,2-Dihydro-1,5-dimethyl-2-pyridylidene)-N'-phenyl-4-morpholinecarboxamidine*

Morpholine (2.70 g) in tetrahydrofuran (30 ml) was added dropwise, at 0°C, with stirring to a solution of phenylisocyanide dichloride (2.70 g) in tetrahydrofuran (40 ml), stirred for one hour then filtered. The filtrate was added to a mixture of 1,5-dimethyl-1,2-dihydro-2-iminopyridine (3.79 g) in tetrahydrofuran (50 ml) methylene chloride (50 ml) and the mixture stirred overnight then filtered. The filtrate was evaporated to dryness, the residue dissolved in dilute hydrochloric acid, washed with diethyl ether (X 1), basified with dilute sodium hydroxide solution and extracted with diethyl ether (X 2). The combined organic extracts were washed with water (X 1), dried over anhydrous magnesium sulphate, filtered and evaporated to dryness. The residue was crystallised from isopropanol to give analytically pure product, mpt 98—9°.

Examples 15 and 16

By use of the appropriate imine and amine, the following compounds have been prepared from phenyl-isocyanaide dichloride by an analogous procedure to that described in Example 14.

Example 15

N,N - diethyl - N' - (1,2 - dihydro - 1 - methyl - 2 - pyridylidene) - N'' - phenylguanidine, mpt 113—4° (petroleum ether, bp 60—80°).

Example 16

N - (1,2 - dihydro - 1 - methyl - 2 - pyridylidene) - N' - phenyl - 4 - thiamorpholinecarboxamidine, mpt 208—210°.

Example 17

*N - (1,2 - Dihydro - 1 - methyl - 2 - pyridylidene) - N' - phenyl - 1 - pyrrolidinecarboxamidine methiodide*

A mixture of N - (1,2 - dihydro - 1 - methyl - 2 - pyridylidene) - N' - phenyl - 1 - pyrrolidinecarboxamidine (1.78 g) and iodomethane (1.30 g) in tetrahydrofuran (25 ml) was allowed to stand for 24 hours. Filtration gave the desired methiodide, mpt 188—9°, of analytical purity.

Biological Data

*Activity on Glucose Tolerance in Fasted Mice*

For this assay mice were fasted for 24 hours before the experiment and then randomised so that each treatment group contained 8 mice. The compounds were dosed orally in 1% aqueous carboxymethyl cellulose (10 ml/kg body weight), and 30 minutes later glucose (1g/kg) was administered by the sub-cutaneous route. Blood samples for glucose analysis were taken from the tail 60 minutes after glucose administration; the results are shown in the table below.

N.B.

A standard system for indicating the significance of results with respect to the controls (dose = zero mmol/kg) which received the 1% aqueous carboxymethyl cellulose vehicle only, is as follows:

** $P < 0.05$
*** $P < 0.01$
**** $P < 0.001$

In the following Table, column A represents the dose of compound (mmol/kg body weight) and column B represents the blood glucose concentration (mmol/litre) 60 minutes after sub-cutaneous glucose load.

TABLE

| Compound | A | B |
|---|---|---|
| Example 3 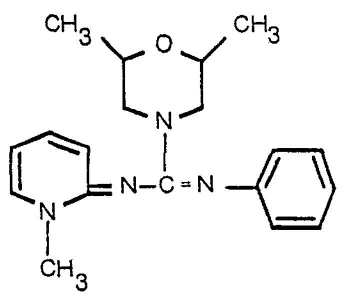 | 0<br>0.2 | 4.88<br>3.48 |
| Example 4 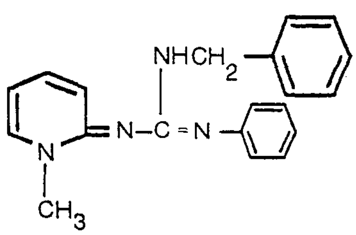 | 0<br>0.2 | 6.67<br>5.36 ** |
| Example 10 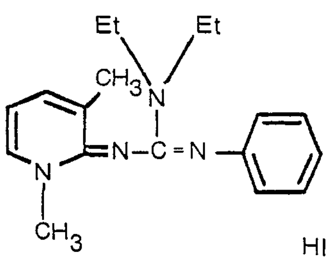 HI Salt | 0<br>0.2 | 8.45<br>5.54 *** |

TABLE (Continued)

| Compound | A | B |
|---|---|---|
| Example 11<br>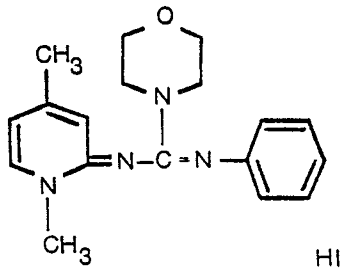<br>HI Salt | 0<br>0.2 | 6.12<br>4.47 ** |
| Example 12<br>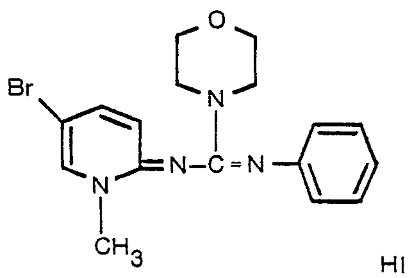<br>HI Salt | 0<br>0.2 | 6.12<br>3.52 *** |
| Example 13<br>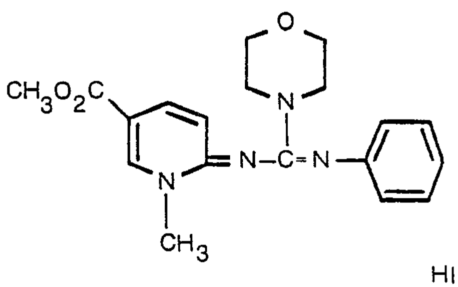<br>HI Salt | 0<br>0.2 | 4.88<br>3.36 ** |
| Example 14<br>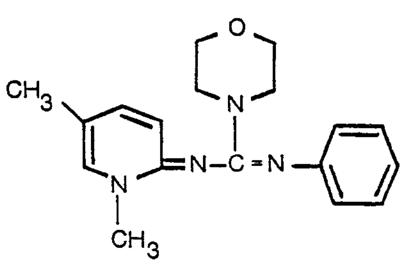 | 0<br>0.2 | 6.42<br>2.53 **** |

## Claims

1. A compound of formula (II) or a pharmaceutically acceptable quaternary ammonium or acid addition salt thereof:

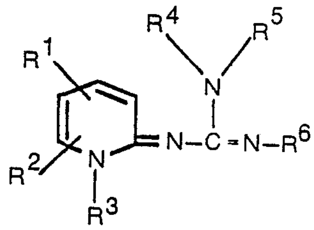

(II)

wherein $R^1$ and $R^2$ are substituents attached to any of the carbon atoms of the dihydropyridine ring and represent hydrogen, $C_{1-6}$ alkyl, halogen, carbo-$C_{1-6}$ alkoxy or carboxy.

$R^3$ represents $C_{1-6}$ alkyl;

$R^4$ represents hydrogen or $C_{1-6}$ alkyl;

$R^5$ represents $C_{1-6}$ alkyl, phenyl optionally substituted with up to 3 groups selected from halogen, $C_{1-6}$ alkyl, and $C_{1-6}$ alkoxy; or benzyl optionally substituted with up to 3 groups selected from halogen, $C_{1-6}$ alkyl and $C_{1-6}$ alkoxy; or $R^4$ and $R^5$ together represent the remaining members of a 5- or 6-membered ring optionally containing an oxygen, sulphur or additional nitrogen atom and being optionally substituted with $C_{1-6}$ alkyl; and

$R^6$ represents $C_{1-6}$ alkyl, phenyl, optionally substituted with up to 3 groups selected from halogen, nitro-, amino-, or trifluoromethyl, $C_{1-6}$ alkyl and $C_{1-6}$ alkoxy; or benzyl optionally substituted with up to 3 groups selected from halogen, $C_{1-6}$ alkyl and $C_{1-6}$ alkoxy, with the proviso that the compounds and acid addition salts thereof wherein, simultaneously, $R^1$ and $R^2$ are both hydrogen, $R^3$ is methyl, $R^4$ is methyl or ethyl, $R^5$ is $C_{1-4}$ alkyl or benzyl,

or 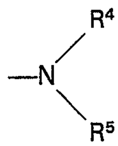 taken together represents 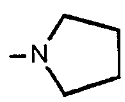, 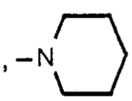, or 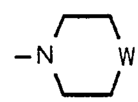

wherein W is oxygen, sulphur or N—$C_{1-4}$ alkyl, are disclaimed.

2. A compound according to claim 1 in which $R^3$ is methyl, ethyl or *n*-propyl.

3. A compound according to claim 1 or claim 2, in which $R^6$ represents phenyl optionally substituted with halogen, $C_{1-6}$ alkyl or nitro.

4. A compound according to any one of claims 1 to 3, in which $R^4$ and $R^5$ together represent a pyrrolidine, piperidine, morpholine or thiamorpholine ring, optionally substituted with $C_{1-6}$ alkyl.

5. A compound according to claim 1, having the formula (III), or a pharmaceutically acceptable quaternary ammonium or acid addition salt thereof:

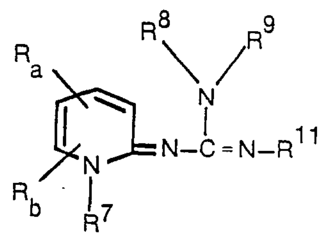

(III)

wherein $R_a$ and $R_b$ are the same as $R^1$ and $R^2$ respectively as defined in formula (II), $R^7$ represents $C_{1-6}$ alkyl;

$R^8$ represents hydrogen or $C_{1-6}$ alkyl;

$R^9$ represents $C_{1-6}$ alkyl or benzyl; or $R^8$ and $R^9$ together form the remaining members of a pyrrolidine or morpholine ring, and

$R^{11}$ represents phenyl optionally substituted with up to 3 groups selected from $C_{1-6}$ alkyl, halogen, nitro or amino.

6. A compound according to claim 5, in which $R^8$ and $R^9$ are both ethyl.

7. A compound according to claim 1 selected from the following, or their pharmaceutically acceptable quaternary ammonium or acid addition salts, N - (1,2 - dihydro - 1 - methyl - 2 -pyridylidene) - N'-phenyl - 4- (2,6 - dimethylmorpholine)carboxamidine, N - benzyl - N' - (1,2 - dihydro - 1 - methyl - 2-pyridylidene) - N'' - phenylguanidine, N,N - diethyl - N' - (1,2 - dihydro - 1,3 - dimethyl - 2 - pyridylidene) - N'' - phenylguanidine, N - (1,2 - dihydro - 1,4 - dimethyl - 2 - pyridylidene) - N' - phenyl - 4-morpholinecarboxamidine, N - (5 - bromo - 1,2 - dihydro - 1 - methyl - 2 - pyridylidene) - N' - phenyl-4 - morpholinecarboxamidine, N - (5 - carbomethoxy - 1,2 - dihydro - 1 - methyl - 2 -pyridylidene) - N'-phenyl - 4 - morpholinecarboxamidine, N - (1,2 - dihydro - 1,5 - dimethyl - 2 - pyridylidene) - N'-phenyl - 4 - morpholinecarboxamidine.

8. A process for the preparation of a compound of formula (II) as defined in claim 1, in which a compound of formula (IV) or a salt thereof:

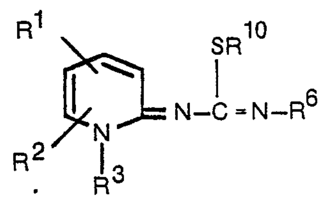

(IV)

wherein $R^1$, $R^2$, $R^3$ and $R^6$ are as defined with respect to formula (II) above, and $R^{10}$ represents $C_{1-6}$ alkyl; is reacted with an amine of formula $R^4R^5NH$, wherein $R^4$ and $R^5$ are as defined with reference to formula (II) above, and thereafter, where desired, a free base of formula (II) so obtained is converted into a pharmaceutically acceptable salt, or a salt of a compound of formula (II) is converted into the free base.

9. A process for the preparation of a compound of formula (II) as defined in claim 1, in which a compound of formula (VII):

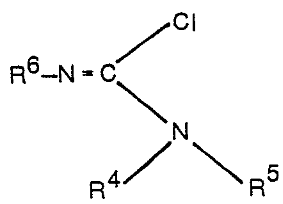

(VII)

wherein R4, $R^5$ and $R^6$ are as defined with respect to formula (II) above; is reacted with an imino compound (V)

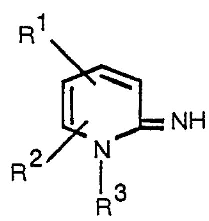

(V)

where $R^1$, $R^2$ and $R^3$ are as defined with repsect to formula (II) above, and thereafter where desired, a free base of formula (II) so obtained is converted into a pharmaceutically acceptable salt, or a salt of a compound of formula (II) is converted into the free base.

10. A pharmaceutical composition which comprises a compound, or a pharmaceutically acceptable quaternary ammonium or acid addition salt thereof, according to any one of claims 1 to 7, together with a pharmaceutical carrier or excipient.

11. A composition according to claim 10 in unit dosage form.

## Revendications

1. Composé de formule (II) ou sel d'ammonium quaternaire ou d'addition avec un acide pharmaceutiquement acceptable de ce composé:

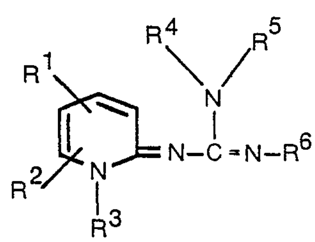

(II)

dans laquelle $R^1$ et $R^2$ sont des substituants fixés sur l'un quelconque des atomes de carbone du cycle

dihydropyridine et représentent de l'hydrogène, un alcoyle en $C_{1-6}$, un halogène, un carbo-alcoxy ($C_{1-6}$) ou un carboxy;

$R^3$ représente un alcoyle en $C_{1-6}$;

$R^4$ représente de l'hydrogène ou un alcoyle en $C_{1-6}$;

$R^5$ représente un groupe alcoyle en $C_{1-6}$, phényle facultativement substitué par un nombre allant jusqu'à 3 groupes choisis par les suivants: halogène, alcoyle en $C_{1-6}$ et alcoxy en $C_{1-6}$; ou benzyle facultativement substitué par un nombre pouvant aller jusqu'à 3 groupes choisis parmi les suivants: halogène, alcoyle en $C_{1-6}$ et alcoxy en $C_{1-6}$; ou bien $R^4$ et $R^5$ représentent ensemble les chaînons restants d'un cycle à 5 ou 6 chaînons contenant facultativement un atome d'oxygène, de soufre ou d'azote additionnel et étant facultativement substitué par un alcoyle en $C_{1-6}$; et

$R^6$ représente en groupe alcoyle en $C_{1-6}$, phényle facultativement substitué par un nombre pouvant aller jusqu'à 3 groupes choisis parmi les suivants: halogène, nitro-, amino- trifluorométhyle, alcoyle en $C_{1-6}$ et alcoxy en $C_{1-6}$; ou benzyle facultativement substitué par un nombre pouvant aller jusqu'à 3 groupes choisis parmi les suivants: halogène, alcoyle en $C_{1-6}$ et alcoxy en $C_{1-6}$, étant entendu que les composés et leurs sels d'additions avec un acide dans lesquels, simultanément, $R^1$ et $R^2$ sont tous deux de l'hydrogène, $R^3$ est un méthyle, $R^4$ est un méthyle ou éthyle, $R^5$ est un alcoyle en $C_{1-4}$ ou benzyl,

ou 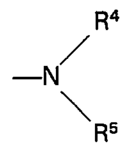 pris ensemble représentent 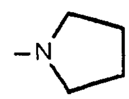, 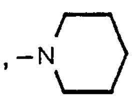, or 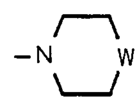

où W est de l'oxygène, du soufre ou un alcoyle en $C_{1-4}$ fixé à l'azote ne sont pas compris dans le cadre de la revendication.

2. Composé suivant la revendication 1, caractérisé en ce que $R^3$ est un groupe méthyle, éthyle ou n-propyle.

3. Composé suivant la revendication 1 ou 2, caractérisé en ce que $R^6$ représente un phényle facultativement substitué par un halogène, alcoyle en $C_{1-6}$ ou nitro.

4. Composé suivant l'une quelconque des revendications 1 à 3, caractérisé en ce que $R^4$ et $R^5$ représentent ensemble un cycle pyrrolidine, pipéridine, morpholine ou thiamorpholine, facultativement substitué par un alcoyle en $C_{1-6}$.

5. Composé suivant la revendication 1, répondant à la formule (III), ou sel d'ammonium quaternaire ou d'addition avec un acide pharmaceutiquement acceptable de celui-ci:

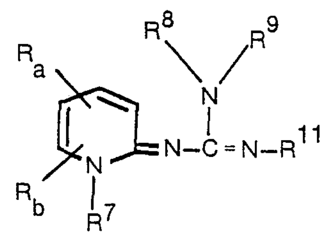

(III)

dans laquelle $R_a$ et $R_b$ sont identiques à $R^1$ et $R^2$ respectivement tels que définis dans la formule (II), $R^7$ représente un alcoyle en $C_{1-6}$;

$R^8$ représente de l'hydrogène ou un alcoyle en $C_{1-6}$;

$R^9$ représente un alcoyle en $C_{1-6}$ ou benzyle; ou bien $R^8$ et $R^9$ forment ensemble les chaînons restants d'un cycle pyrrolidine ou morpholine, et

$R^{11}$ représente un phényle facultativement substitué par un nombre pouvant aller jusqu'à 3 groupes choisis parmi les suivants: alcoyle en $C_{1-6}$, halogène, nitro ou amino.

6. Composé suivant la revendication 5, caractérisé en ce que $R^8$ et $R^9$ sont tous deux un groupe éthyle.

7. Composé suivant la revendication 1, choisi parmi les suivants, ou sels d'ammonium quaternaire ou d'addition avec un acide pharmaceutiquement acceptables: N - (1,2 - dihydro - 1 - méthyl - 2-pyridylidène) - N' - phényl - 4 - (2,6 - diméthylmorpholine)carboxamidine, N - benzyl - N' - (1,2 - dihydro 1 - méthyl - 2 - pyridylidène) - N'' - phénylguanidine, N,N - diéthyl - N' - (1,2 - dihydro - 1,3 - diméthyl-2 - pyridylidène) - N'' - phénylguanidine, N - (1,2 - dihydro - 1,4 - diméthyl - 2 - pyridylidène) - N'-phényl - 4 - morpholinecarboxamidine, N - (5 - bromo - 1,2 - dihydro - 1 -méthyl - 2 - pyridylidène) - N'-phényl - 4 - morpholinecarboxamidine, N - (5 - carbométhoxy - 1,2 - dihydro - 1 - méthyl - 2 - pyridylidène) - N' - phényl - 4 - morpholinecarboxamidine, N - (1,2 - dihydro - 1,5 - diméthyl - 2 - pyridylidène) - N' - phényl - 4 - morpholinecarboxamidine.

8. Procédé pour la préparation d'un composé de formule (II) tel que défini dans la revendication 1, selon lequel on fait réagir un composé de formule (IV) ou un sel de celui-ci:

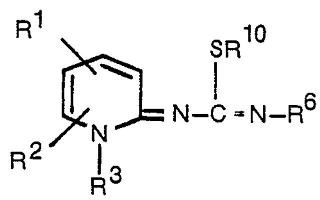

(IV)

dans laquelle $R^1$, $R^2$, $R^3$ et $R^6$ sont tels que définis dans le cas de la formule (II) ci-dessus, et $R^{10}$ représante un alcoyle en $C_{1-6}$, avec un amine de formule $R^4R^5NH$ dans laquelle $R^4$ et $R^5$ sont tels que définis dans le cas de la formule (II) ci-dessus, puis si désiré on convertit une base libre de formule (II) ainsi obtenue en un sel pharmaceutiquement acceptable, ou bien on convertit un sel d'un composé de formule (II) en base libre.

9. Procédé pour la préparation d'un composé de formule (II) suivant la revendication 1, selon lequel on fait réagir un composé de formule (VII):

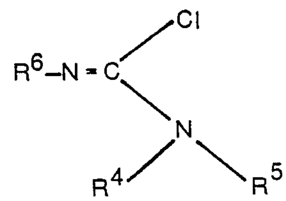

(VII)

dans laquelle $R^4$, $R^5$ et $R^6$ sont tels que définis dans le cas de la formule (II) ci-dessus, avec un composé imino (V):

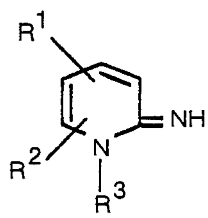

(V)

dans laquelle $R^1$, $R^2$ et $R^3$ sont tels que définis dans le cas de la formule (II) ci-dessus, puis si désiré on convertit une base libre de formule (II) ainsi obtenue en un sel pharmaceutiquement acceptable, ou bien on convertit un sel d'un composé de formule (II) en base libre.

10. Composition pharmaceutique comportant un composé ou un sel d'ammonium quaternaire ou d'addition avec un acide pharmaceutiquement acceptable suivant l'une quelconque des revendications 1 à 7, conjointement à un véhicule ou excipient pharmaceutique.

11. Composition suivant la revendication 10, sous forme posologique unitaire.

**Patentansprüche**

1. Eine Verbindung der Formel (II) oder ein pharmazeutisch annehmbares quaternäres Ammonium- oder Säureadditionssalz derselben:

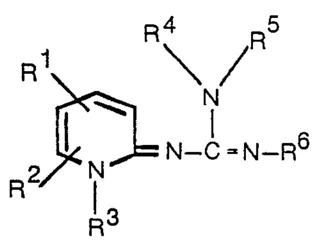

(II)

wobei $R^1$ und $R^2$ an irgendwelche der Kohlenstoffatome des Dihydropyridinringes gebundene Substituenten sind und Wasserstoff, $C_{1-6}$-Alkyl, Halogen, Carbo-$C_{1-6}$-Alkoxy oder Carboxy darstellen;

$R^3$ $C_{1-6}$-Alkyl ist,

$R^4$ Wasserstoff oder $C_{1-6}$-Alkyl ist,

$R^5$ $C_{1-6}$-Alkyl, Phenyl, gewünschtenfalls substituiert mit bis zu 3 Gruppen, ausgewählt aus Halogen, $C_{1-6}$-Alkyl und $C_{1-6}$-Alkoxy, oder Benzyl, gewünschtenfalls substituiert mit bis zu 3 Gruppen, ausgewählt aus Halogen, $C_{1-6}$-Alkyl und $C_{1-6}$-Alkoxy, darstellt, oder $R^4$ und $R^5$ zusammen die Restglieder eines 5- oder 6- gliedrigen Ringes, der gewünschtenfalls ein Sauerstoff- Schwefel- oder zusätzliches Stickstoffatom enthält und gewünschtenfalls mit $C_{1-6}$-Alkyl substituiert ist, darstellen und

$R^6$ $C_{1-6}$-Alkyl, Phenyl, gewünschtenfalls substituiert mit bis zu 3 Gruppen, ausgewählt aus Halogen, Nitro, Amino, oder Trifluormethyl, $C_{1-6}$-Alkyl und $C_{1-6}$-Alkoxy, oder Benzyl, gewünschtenfalls substituiert mit bis zu 3 Gruppen, ausgewählt aus Halogen, $C_{1-6}$-Alkyl und $C_{1-6}$-Alkoxy, darstellt, mit der Maßgabe, daß die Verbindungen und Säureadditionssalze derselbe, in welchen gleichzeitig $R^1$ und $R^2$ Wasserstoff sind, $R^3$ Methyl ist, $R^4$ Methyl oder Äthyl ist, $R^5$ ein $C_{1-4}$-Alkyl oder Benzyl ist,

oder 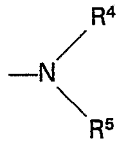 zusammen 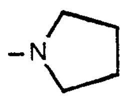 , 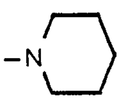 , or 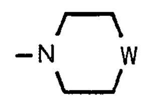

darstellen, wobei W Sauerstoff, Schwefel oder N-$C_{1-4}$-Alkyl ist, nicht beansprucht werden.

2. Eine Verbindung gemäß Anspruch 1, in welcher $R^3$ Methyl, Äthyl oder n-Propyl ist.

3. Eine Verbindung gemäß Anspruch 1 oder 2, in welcher $R^6$ Phenyl, gewünschtenfalls substituiert mit Halogen, $C_{1-6}$-Alkyl oder Nitro, darstellt.

4. Eine Verbindung gemäß irgendeinem der Ansprüche 1 bis 3, in welcher $R^4$ und $R^5$ zusammen einen Pyrrolidin-, Piperidin-, Mopholine- oder Thiamorpholinring, gewünschtenfalls substituiert mit $C_{1-6}$-Alkyl, darstellen.

5. Eine Verbindung gemäß Anspruch 1 der Formel (III), oder ein pharmazeutisch annehmbares quaternäres Ammonium- oder Säureadditionssalz derselben:

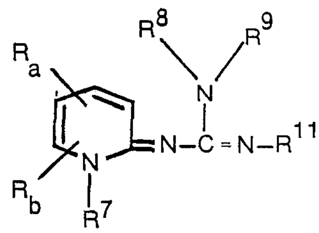

(III)

wobei $R_a$ und $R_b$ entsprechend wie $R^1$ und $R^2$ in Formel (II) definiert sind, $R^7$ $C_{1-6}$-Alkyl ist,

$R^8$ Wasserstoff oder $C_{1-6}$-Alkyl ist,

$R^9$ $C_{1-6}$-Alkyl oder Benzyl darstellt, oder $R^8$ und $R^9$ zusammen die Restglieder eines Pyrrolidin- oder Morpholinringes bilden, und

$R^{11}$ Phenyl, gewünschtenfalls substituiert mit bis zu 3 Gruppen, ausgewählt aus Halogen, Nitro oder Amino, darstellt.

6. Eine Verbindung gemäß Anspruch 5, in welcher $R^8$ und $R^9$ beide Äthyl sind.

7. Eine Verbindung gemäß Anspruch 1, ausgewählt aus den folgenden Verbindungen oder deren pharmazeutisch annehmbaren quaternänen Ammonium- oder Säureadditionssalzen: N - (1,2-Dihydro - 1 - methyl - 2 - pyridyliden) - N' - phenyl - 4 - (2,6 - dimethylmorpholin)carboxamidin, N-Benzyl - N' - (1,2 - dihydro - 1 - methyl - 2 - pyridyliden) - N'' - phenylguanidin, N,N - Diäthyl - N' - (1,2-dihydro - 1,3 - dimethyl - 2 - pyridyliden) - N'' - phenylguanidin, N - (1,2 - Dihydro - 1,4 - dimethyl - 2-pyridyliden) - N' - phenyl - 4 - morpholincarboxamidin, N - (5 - Brom - 1,2 - dihydro - 1 - methyl - 2-pyridyliden) - N' - phenyl - 4 - morpholincarboxamidin, N - (5 - Carbomethoxy - 1,2 - dihydro - 1-methyl - 2 - pyridyliden) - N' - phenyl - 4 - morpholincarboxamidin, N - (1,2 - Dihydro - 1,5 - dimethyl - 2 pyridyliden) - N' - phenyl - 4 - morpholin-carboxamidin.

8. Eine Verfahren zur Herstellung einer Verbindung der Formel (II) wie in Anspruch 1 definiert, in welchem eine Verbindung der Formel (IV) oder ein Salz derselben:

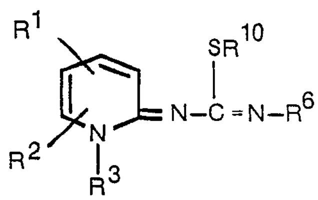

(IV)

wobei $R^1$, $R^2$, $R^3$ und $R^6$ wie in bezug auf Formel (II) definiert sind, und $R^{10}$ $C_{1-6}$-Alkyl ist, mit einem Amin der Formel $R^4R^5NH$ umgesetzt wird, wobei $R^4$ und $R^5$ wie in bezug auf Formel (II) definiert sind, und anschließend, falls erwünscht, eine so erhaltene freie Base der Formel (II) in ein pharmazeutisch annehmbares Salz umgewandelt wird, oder ein Salz einer Verbindung der Formel (II) in die freie Base umgewandelt wird.

9. Eine Verfahren zur Herstellung einer Verbindung der Formel (II) wie in Anspruch 1 definiert, in welchem eine Verbindung der Formel (VII):

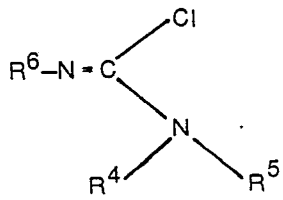

(VII)

wobei $R^4$, $R^5$ und $R^6$ wie in bezug auf Formel (II) definiert sind, mit einer Iminoverbindung (V) umgesetzt wird

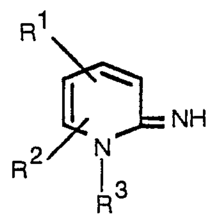

(V)

wobei $R^1$, $R^2$ und $R^3$ wie in bezug auf Formel (II) definiert sind, und anschließend, falls gewünscht, eine so erhaltene freie Base der Formel (II) in ein pharmazeutisch annehmbares Salz, oder ein Salz der Verbindung der Formel (II) in dioe freie Base umgewandelt wird.

10. Eine pharmazeutische Zusammensetzung, welche eine Verbindung gemäß irgendeinem der Ansprüche 1 bis 7 oder ein pharmazeutisch annehmbares quaternäres Ammonium- oder Säureadditionssalz derselben, zusammen mit einem pharmazeutischen Träger bzw. Arzneimittelträger umfaßt.

11. Eine Verbindung gemäß Anspruch 10 in Einzeldosisform.